# EUROPÄISCHE PATENTSCHRIFT

(11) **EP 2 353 516 B1**
(45) Veröffentlichungstag und Bekanntmachung des Hinweises auf die Patenterteilung: **19.12.2012**
(21) Anmeldenummer: 11000077.5
(22) Anmeldetag: 07.01.2011
(51) Int. Cl.: A61B 17/062, A61B 17/06, A61B 17/29

(54) **Medizinisches Greifinstrument**
Medical gripping instrument
Instrument de prise médical

(30) Priorität: 29.01.2010 DE 102010006201
(43) Veröffentlichungstag der Anmeldung: 10.08.2011
(73) Patentinhaber: Richard Wolf GmbH, 75438 Knittlingen (DE)
(72) Erfinder: Dingler, Andreas, 75217 Birkenfeld (DE)
(74) Vertreter: Hemmer, Arnd

(56) Entgegenhaltungen:
- DE-A1- 10 031 773
- DE-A1- 19 537 320
- DE-A1- 19 547 188

## Beschreibung

Die Erfindung betrifft ein medizinisches Greifelement gemäß dem Oberbegriff des Anspruchs 1. Medizinische Greifinstrumente, insbesondere endoskopische Greifinstrumente sind für verschiedene Zwecke bekannt und weisen bewegliche Maulteile zum Greifen von Gegenständen oder Gewebeteilen bei Operationen auf. Beispielsweise sind medizinische Greifinstrumente in Form von Nadelübernahmeinstrumenten bekannt, welche bei endoskopischen Operationen für Näharbeiten eingesetzt werden, um Nadeln zu ergreifen und zum Vernähen von Wunden zu führen. Bei den bekannten Nadelübernahmeinstrumenten ist meist eine Sperre vorgesehen, welche bei jedem Greifen ein- und ausgeschaltet werden muss. Dies macht die Handhabung umständlich. Im Übrigen sind derartige Instrumente von ihrem Aufbau her aufwendig und somit teuer.

DE 195 37 320 A1 sowie DE 131 773 A1 offenbaren gelenklose medizinische Zangen, bei welchen die Maulteile durch elastische Verformung ausgelenkt werden. Dazu weist jedes Maulteil proximalseitig zwei lateral beabstandete flexible Verbindungsteile auf, wobei an den innen gelegenen Verbindungsteilen ein Zug- / Druckelement angreift, während die außen gelegenen Verbindungsteile allein eine Gelenkfunktion durch elastische Verformung haben. Durch axiale Bewegung des Zug- /Druckelementes kann das Maulteil geöffnet oder geschlossen werden. Auch dieses Instrument benötigt entweder eine konstante Haltekraft, um einen Gegenstand zwischen den Maulteilen zu fixieren oder eine Sperre, welche die Maulteile im geschlossenen Zustand fixiert. Dies macht die Handhabung umständlich.

Es ist Aufgabe der Erfindung, ein verbessertes medizinisches Greifinstrument, insbesondere ein endoskopisches medizinisches Greifinstrument, wie ein Nadelübernahmeinstrument bereitzustellen, welches ein sicheres Halten beispielsweise eines Fadens oder einer Nadel ermöglicht, ohne eine Sperre betätigen zu müssen. Darüber hinaus soll das Instrument von seinem Aufbau her möglichst einfach und kostengünstig zu fertigen sein.

Diese Aufgabe wird durch ein medizinisches Greifinstrument mit den im Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße medizinische Greifinstrument ist insbesondere ein endoskopisches medizinisches Greifinstrument, welches bei endoskopischen Operationen für Greif- und Handhabungsaufgaben im Operationsgebiet Verwendung findet. Besonders bevorzugt handelt es sich bei dem medizinischen Greifinstrument um ein Übernahmeinstrument, wie ein Nadelübernahmeinstrument, welches zum Greifen einer Nadel oder eines Fadens beim Vernähen Verwendung findet.

Das erfindungsgemäße Greifinstrument weist an seinem distalen Ende ein Maul auf, welches aus zwei Maulteilen gebildet wird, welche jeweils einen Greifabschnitt aufweisen. Dabei bildet jeweils der distale Abschnitt des Maulteiles den Greifabschnitt. Die beiden Greifabschnitte liegen einander gegenüber, sodass zwischen den beiden Greifabschnitten Dinge, wie beispielsweise Nadel und Faden oder Gewebeabschnitte, gegriffen werden können. Erfindungsgemäß weist zumindest eines der Maulteile neben dem Greifabschnitt einen proximalseitig des Greifabschnittes gelegenen Halteabschnitt auf. Dabei sind der Greifabschnitt und der zugehörige Halteabschnitt des Maulteiles vorzugsweise fest verbunden und insbesondere sind der Greifabschnitt und der Halteabschnitt des Maulteiles bevorzugt einteilig oder einstückig ausgebildet. Dies ermöglicht eine kostengünstige Fertigung und Montage, da die Zahl der erforderlichen Einzelteile gering gehalten wird.

Der Halteabschnitt ist elastisch verformbar ausgebildet. Dabei ist der Halteabschnitt zumindest eines der Maulteile derart elastisch verformbar, dass eine Auslenkung des zugehörigen mit diesem Halteabschnitt verbundenen Greifabschnittes durch die elastische Verformung möglich ist. Der elastisch verformbare bzw. elastisch biegbare Halteabschnitt des Maulteiles übernimmt somit eine Gelenkfunktion, sodass es möglich ist, das Maul des Greifinstrumentes gelenklos auszubilden. Insofern wird es möglich, allein durch elastische Verformung des zumindest einen Halteabschnittes die beiden Greifabschnitte relativ aufeinander und relativ voneinander weg zu bewegen. D. h. das Maul, welches zwischen den Greifabschnitten gebildet wird, kann durch elastische Verformung zumindest eines Halteabschnittes geöffnet und geschlossen werden. Die gelenklose Ausgestaltung vereinfacht den Aufbau des Instrumentes, da hierdurch wiederum die Zahl der erforderlichen Einzelteile und Montageschritte verringert werden kann. Darüber hinaus ist ein solches Instrument aufgrund der verringerten Zahl von Einzelteilen und damit der verringerten Zahl von Spalten zwischen den einzelnen Einzelteilen einfacher zu reinigen und zu desinfizieren.

Die Greifabschnitte der beiden Maulteile liegen in einer Ruhelage aneinander an und sind durch elastische Verformung zumindest eines Halteabschnittes auseinander bewegbar. Durch diese Verformung des Halteabschnittes wird das Maul zwischen den Greifabschnitten geöffnet. Dies ist von Vorteil für ein Nadelübemahmeinstrument. Dadurch, dass das Instrument in seiner Ruhelage geschlossen ist, wird eine einmal aufgenommene Nadel oder ein einmal aufgenommener Faden somit in der Ruhelage des Instrumentes zwischen den beiden Greifabschnitten gehalten.

Gemäß einer bevorzugten Ausführungsform der Erfindung weisen beide Maulteile neben einem Greifabschnitt jeweils einen proximalen Halteabschnitt auf, welcher derart elastisch verformbar ist, dass die Greifabschnitte der beiden Maulteile durch elastische Verformung der beiden Halteabschnitte aufeinander zu und voneinander weg bewegbar sind. Auch hier ist es bevorzugt, das der Greifabschnitt eines Maulteiles und der proximolseitig angrenzende Halteabschnitt fest miteinander verbunden sind, bevorzugt einstückig oder einteilig ausgebildet sind, um die Zahl der erforderlichen Einzelteile zu reduzieren. Bevorzugt sind die beiden Maulteile symmetrisch zueinander bezogen auf die Instrumentenlängsachse ausgebildet, sodass sie zum Öffnen und Schließen des Maules symmetrisch auseinander bewegt bzw. aufeinander zu bewegt werden. Auch bei dieser Ausführungsform können die Maulteile so ausgebildet sein, dass sie in ihrer Ruhelage geschlossen sind und durch Verformung der Halteabschnitte auseinander bewegt werden. Auch die umgekehrte Ausgestaltung ist denkbar, bei welcher das Maul in seiner Ruhelage geöffnet ist und durch Verformung der Halteabschnitte geschlossen wird.

Aufgrund der elastischen Verformbarkeit der Halteabschnitte wird erreicht, dass diese durch Einwirken einer äußeren Kraft verformt werden können, um das Maul zu öffnen oder zu schließen, je nach Ausgestaltung des Maules. Sobald diese äußere Kraft gelöst wird, bewegen sich die Halteabschnitte und damit die mit Ihnen verbundenen Greifabschnitte aufgrund der Elastizität des Materiales der Halteabschnitte in ihre Ausgangs- bzw. Ruhelage zurück. So übernimmt die elastische Verformbarkeit des Halteabschnittes nicht nur die Gelenkfunktion, sondern bildet gleichzeitig eine Rückstellfeder.

Erfindungsgemäß ist in der Ruhelage zumindest ein Halteabschnitt eines Maulteiles bereits derart elastisch verformt, dass er eine Federkraft erzeugt, welche die Greifabschnitte der beiden Maulteile in Anlage hält. Bevorzugt weisen auch bei dieser Ausgestaltung beide Maulteile einen entsprechend elastisch verformbaren Halteabschnitt auf und beide Halteabschnitte sind in ihrer Ruhelage elastisch verformt, sodass sie gegeneinander vorgespannt sind und die beiden Gelenkteile unter Vorspannung in Anlage halten. Auf diese Weise kann in der Ruhelage eine Haltekraft bzw. Klemmkraft zwischen den beiden Greifabschnitten erzeugt werden, beispielsweise um einen Faden oder eine Nadel in der Ruhelage sicher halten zu können. So kann auf aufwendige Sperrmechanismen verzichtet werden. Insgesamt sind die Maulteile bzw. deren Halteabschnitte somit nach Art einer Blattfeder ausgestaltet. Dabei sind die Maulteile so ausgestaltet, dass sie in ihrem Ausgangszustand d. h., wenn sie nicht montiert sind, so geformt bzw. gekrümmt sind, dass der Greifabschnitt des Maulteiles sich über die spätere Anlageebene mit dem zweiten Maulteil hinaus erstreckt, sodass er an der entgegengesetzten Seite dieser Anlageebene als sein zugehöriger Halteabschnitt gelegen ist. Wenn eines oder beide Maulteile entsprechend ausgebildet sind, sodass ihre Greifabschnitte aufeinander zu ausgerichtet sind, hat dies den Effekt, dass, wenn die beiden Maulteile im Instrument montiert sind, die beiden Greifabschnitte so zur Anlage kommen, dass die Halteabschnitte um ein gewisses Maß elastisch verformt werden, sodass die beiden Greifabschnitte jeweils an derselben Seite der Anlageebene wie ihr zugehöriger Halteabschnitt zur Anlage kommen und die Anlageebene der beiden Greifabschnitte bevorzugt genau auf der Instrumentenlängsachse gelegen ist. Auf diese Weise wird eine Vorspannung in den Halteabschnitten erzeugt, welche eine Haltekraft zwischen den beiden Greifabschnitten bewirkt. Zum Öffnen des Zangenmaules müssen die Halteabschnitte weiter verformt werden, sodass die Greifabschnitte jeweils von der Mittelebene bzw. Anlageebene weg bewegt werden. Es ist zu verstehen, dass entsprechend auch ein gewinkeltes Maul gebildet werden kann. Bei diesem erstreckt sich die Anlageebene gewinkelt zur Instrumentenlöngsachse.

Zum Öffnen des Mauls ist ein keilförmiges Betätigungselement vorhanden, welches linear in Richtung seiner Spitze derart zwischen die Maulteile bewegbar ist, dass die Greifabschnitte der beiden Maulteile unter elastischer Biegung zumindest eines Halteabschnittes eines Maulteiles auseinander bewegt werden. Bei dieser Ausgestaltung befinden sich somit die Greifabschnitte der Maulteile in ihrer Ruhelage in Anlage, vorzugsweise in vorgespannter Anlage, bei welcher zumindest ein Halteabschnitt eine Federkraft erzeugt, durch welche die beiden Greifabschnitte aneinander gedrückt werden. Das keilförmige Betätigungselement ist so zwischen die Maulteile, insbesondere deren Halteabschnitte bewegbar, dass es ausgehend von seiner Spitze bei weiterer linearer Bewegung aufgrund seiner Keilform die beiden Maulteile und damit deren Greifabschnitte auseinander drückt, sodass das zwischen den Greifabschnitten gebildete Maul geöffnet wird.

Um diese Bewegung zu erzeugen, weist zumindest eines der Maulteile, welches verform- bzw. auslenkbar ist, eine Betätigungsfläche auf, mit welcher das Betätigungselement zur Bewegung des Greifabschnittes des Maulteiles in Kontakt bringbar ist. In der Ruhelage ist der Abstand dieser Betätigungsfläche von der Instrumentenlöngsachse geringer als der weiteste Abstand der Keilfläche von dieser Achse. Die schräge Keilfläche des Betätigungselementes gleitet bei dessen Bewegung in axialer Richtung auf dieser Betätigungsfläche. Um die Reibung möglichst gering zu halten, kann die Betätigungsfläche möglichst klein ausgebildet werden. Idealerweise ist die Fläche so klein, dass nur ein Punkt- oder Linienkontakt gebildet wird. Beispielsweise kann die Betätigungsfläche nur von einer Kante im Übergangsbereich zwischen Halteabschnitt und Greifabschnitt des auslenkbaren Maulteiles bewegt sein. Bei dieser Ausgestaltung kann im Halteabschnitt eine Ausnehmung ausgebildet sein, in welcher das Betätigungselement in seiner Ruhelage vollständig aufgenommen ist. Bei linearem Vorschub kommt es dann mit seiner Keilfläche an einer Endkante der Ausnehmung, welche im Übergangsbereich zu dem Greifabschnitt gelegen ist, zur Anlage, wobei diese Kante die Betätigungsfläche bildet. Vorzugsweise ist diese Betätigungsfläche gerundet ausgebildet.

Wenn beide Maulteile entsprechend auslenkbar sind, ist vorzugsweise an jedem der Maulteile eine solche Betätigungsfläche vorgesehen, wobei diese einander gegenüberliegen und gegebenenfalls entgegengesetzt zueinander geneigt oder gebogen sind, um mit zwei zueinander entgegengesetzt geneigten Keilflächen des Betätigungselementes zur Anlage zu kommen.

Zum Schließen des Mauls des Instrumentes wird das Betätigungselement wieder in linearer Richtung zurück bewegt, wobei die Maulteile aufgrund der elastischen Auslenkung der Halteabschnitte sich dann selbsttätig wieder in ihre Ruhelage zurück bewegen. Die elastische Auslenkung der Halteabschnitte bewirkt eine elastische Spannung in den Halteabschnitten, welche dafür sorgt, dass die Halteabschnitte bestrebt sind, selbsttätig in ihre Ausgangslage zurück zu kehren.

Zur Betätigung des von den Greifabschnitten gebildeten Instrumentenmauls ist weiter bevorzugt zur Bewegung des Greifabschnittes zumindest eines der Maulteile eine in Richtung der Instrumentenlängsachse bewegliche Betätigungsstange vorhanden, welche an ihrem proximalen Ende mit einer Handhabe verbunden ist. Die Handhabe kann direkt oder indirekt mit der Betätigungsstange verbunden bzw. gekoppelt sein, z.B. über ein Blattfederelement, wie es nachfolgend beschrieben wird. Diese Betätigungsstange erstreckt sich somit entlang der Instrumentenlängsachse vom proximalen Ende des Instrumentes, an welchem die Handhabe gelegen ist, zum distalen Ende des Instrumentes, an welchem durch die beiden Greifabschnitte das Instrumentenmaul gebildet ist. Durch lineares Verschieben der Betätigungsstange wird zumindest einer, bevorzugt werden beide Greifabschnitte bewegt. Dazu ist bevorzugt ein Betätigungselement gemäß der vorangehenden Beschreibung am distalen Ende der Betätigungsstange angeordnet, sodass durch lineares Verschieben der Betätigungsstange in distaler Richtung das Betätigungselement weiter zwischen die beiden Maulteile bewegt wird, um diese quer zur Bewegungsrichtung der Betätigungsstange auseinander zu bewegen. Zum Schließen des Mauls wird die Betätigungsstange und damit das mit ihrem distalen Ende verbundene Betätigungselement wieder in proximaler Richtung zurück bewegt, wobei sich das Maul durch Bewegung der Maulteile aufgrund der elastischen Rückstellkräfte in den Halteabschnitten selbsttätig schließt.

Gemäß einer weiteren bevorzugten Ausführungsform weist das erfindungsgemäße Greifinstrument eine Handhabe auf, welche zumindest einen Griffschenkel hat, der an seinem distalen Ende gelenkig gelagert und an seinem proximalen Ende mit einem Blattfederelement verbunden ist, welches sich ausgehend von dem proximalen Ende des Griffschenkels distalwärts erstreckt und mit seinem distalen Ende beabstandet von dem Griffschenkel mit dem proximalen Ende einer Betätigungsstange verbunden ist. Das Blattfederelement liegt im Inneren der Handhabe und ist dabei am proximalen Ende des Griffschenkels vorzugsweise fest angebracht und erstreckt sich von dort aus derart in distaler Richtung, dass es sich entlang seiner Erstreckung immer weiter von dem Griffschenkel entfernt. An seinem distalen Ende ist das Blattfederelement dann direkt oder indirekt mit dem proximalen Ende der Betätigungsstange verbunden. Dabei ist das Blattfederelement mit dem proximalen Ende der Betätigungsstange vorzugsweise gelenkig verbunden. Das Blattfederelement übernimmt zwei Funktionen. Zum einen überträgt es die Bewegung des Griffschenkels auf die Betätigungsstange, sodass bei Verschwenken des Griffschenkels die Betätigungsstange linear, d. h. in Richtung der Instrumentenlängsachse bewegt wird. Vorzugsweise wird beim Verschwenken des Griffschenkels nach innen, d. h. zur Instrumentenlängsachse hin, das Federelement so bewegt und verformt, dass der Verbindungspunkt mit der Betätigungsstange distalwärts verschoben wird und somit auch die Betätigungsstange in distaler Richtung bewegt wird. Zum anderen übernimmt das Federelement gleichzeitig die Funktion einer Rückstellfeder. Zur Bewegung der Betätigungsstange wird das Blattfederelement bei Verschwenken des Griffschenkels elastisch gebogen bzw. verformt. Aufgrund dieser elastischen Verformung des Blattfederelementes wird eine Rückstellkraft erzeugt, welche beim Lösen des Druckes auf den Griffschenkel diesen in seine Ausgangslage zurück bewegt, wobei gleichzeitig auch die Betätigungsstange in ihre Ausgangslage zurück bewegt wird.

Bevorzugt weist die Handhabe zwei derartige Griffschenkel mit jeweils einem solchen Blattfederelement auf. Die Blattfederelemente liegen zwischen den beiden Griffschenkeln im Inneren der Handhabe. Die beiden Griffschenkel sind an einem gemeinsamen oder einem jeweils separaten Anlenkungspunkt gelenkig gelagert. Dabei liegen die Anlenkungspunke vorzugsweise symmetrisch zur Instrumentenlängsachse, insbesondere ist die gesamte Handhabe bevorzugt spiegelsymmetrisch zur Instrumentenlängsachse ausgebildet. Entsprechend sind auch zwei Blattfederelemente bei dieser Ausführungsform vorgesehen, welche bevorzugt in einem gemeinsamen Anlenkungspunkt gelenkig mit dem proximalen Ende der Betätigungsstange verbunden sind. Dieser Anlenkungspunkt liegt dabei bevorzugt auf der Symmetrielinie der Handhabe, d. h. bevorzugt auf der Instrumentenlängsachse. Die Blattfederelemente sind in ihrer Ruhelage gekrümmt ausgebildet, sodass sie sich ausgehend vom proximalen Ende, welches beabstandet zur Instrumentenlängsachse gelegen ist, zur Instrumentenlängsachse hin krümmen und dabei von der Innenseite des zugehörigen Griffschenkels weiter entfernen. Wenn die Griffschenkel aufeinander zu bewegt werden, führt dies zu einer Verbiegung der Blattfederelemente, sodass diese eine gestrecktere Gestalt annehmen, wodurch sich der Anlenkungspunkt an der Betätigungsstange distalwärts verlagert.

Weiter bevorzugt ist die Anbindung des Blattfederelementes an das proximale Ende der Betätigungsstange in proximaler Richtung von der gelenkigen Lagerung des Griffschenkels beabstandet. Griffschenkel und Blattfederelemente schwenken somit um unterschiedliche Anlenkungspunkte. Dabei ist das Blattfederelement in Richtung der Instrumentenlängsachse kürzer ausgebildet als die Griffschenkel, sodass es beim Druck auf die Griffschenkel zu der oben beschriebenen Verbiegung bzw. Streckung des bzw. der Blattfederelemente kommt und die Betätigungsstange axial verlagert wird.

Nachfolgend wird die Erfindung beispielhaft anhand der beigefügten Figuren beschrieben. In diesen zeigt:
- Fig. 1: die Gesamtansicht eines medizinischen Greifinstrumentes in Form eines Nadelübernahmeinstrumentes,
- Fig. 2: eine vergrößerte Ansicht des distalen Endes des Instrumentes gemäß Fig. 1 mit geöffnetem Maul,
- Fig.3: eine vergrößerte Detailansicht des geöffneten Maules gemäß Fig. 1 und 2,
- Fig. 4: die Ansicht gemäß Fig. 2 in einem teilweisen Längsschnitt,
- Fig. 5: einen Querschnitt entlang der Linie V - V in Fig. 2, und
- Fig. 6: einen Querschnitt entlang der Linie VI - VI in Fig. 2.

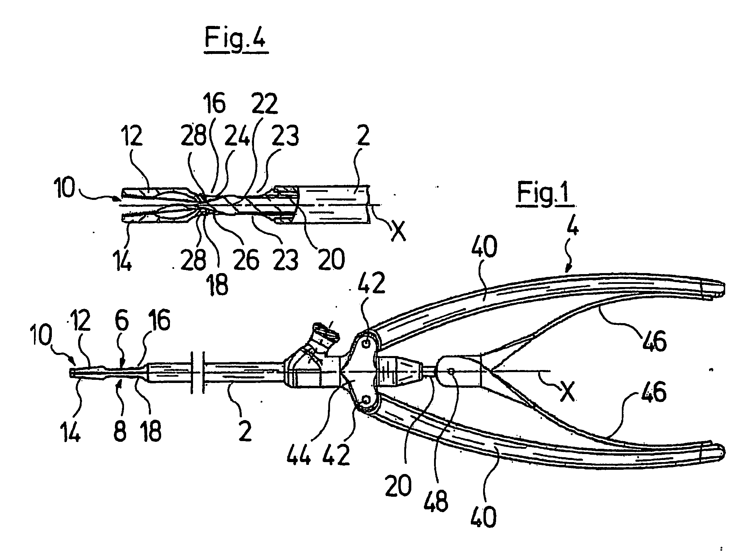

Das in den Figuren gezeigte medizinische Greifinstrument ist ein Nadelübernahmeinstrument. Dieses weist einen Schaft 2 auf, an dessen proximalen Ende eine Handhabe 4 und an dessen distalen Ende eine aus zwei Maulteilen 6 und 8 gebildetes Maul gelegen ist. In dem in Fig. 1 gezeigten Ruhezustand liegen die Maulteile 6 und 8 mit ihren Greifabschnitten 12 und 14 aneinander an, sodass das Maul 10 geschlossen ist.

Erfindungsgemäß ist das Maul 10 bei dem gezeigten Instrument gelenklos ausgebildet. D. h. es gibt kein Gelenk, in welchem starre Maulteile schwenkbar angelenkt sind. Vielmehr sind hier die Maulteile 6 und 8 aus einem federnden bzw. elastischen Material, vorzugsweise Metall, ausgebildet und die Auslenkung der Maulteile bzw. insbesondere deren Greifabschnitte 12 und 14 zum Öffnen des Zangenmaules erfolgt durch elastische Verformung der Maulteile 6 und 8.

Die Maulteile 6 und 8 weisen an ihrem distalen Ende jeweils einen Greifabschnitt 12, 14 auf. Proximalseitig schließt an diese Greifabschnitte 12 und 14 jeweils ein Halteabschnitt 16, 18 an. Der Halteabschnitt 16 ist einstückig mit dem Greifabschnitt 12 und der Halteabschnitt 18 ist einstückig mit dem Greifabschnitt 14 ausgebildet. Die Halteabschnitte 16 und 18 sind an ihrem proximalen Ende jeweils fest mit dem distalen Ende des Schaftes 2 verbunden.

In der in Fig. 1 gezeigten Ruhelage liegen die beiden Maulteile 6 und 8 mit den einander zugewandten Innenflächen parallel zur Instrumentenlängsachse X aneinander an. Um in diesem Zustand eine feste Anlage der Greifabschnitte 12 und 14 zu gewährleisten, können die Maulteile 6, 8 jeweils so geformt sein, dass sie in ihrer Ausgangsform, in welcher sie nicht an dem Schaft 2 montiert sind, jeweils so geformt sind, dass die Halteabschnitte 16, 18 und ggf. die Greifabschnitte 12, 14 gekrümmt ausgebildet sind, sodass die Innenfläche des Maulteiles 6, 8, welche zur Anlage an dem gegenüberliegenden Maulteil vorgesehen ist, in dieser Ausgangslage im Wesentlichen eine konkave Krümmung aufweist. Hierdurch wird erreicht, dass bei der Montage der beiden Maulteile 6 und 8 aneinander bereits eine elastische Verformung der Halteabschnitte 16, 18 erforderlich ist, um die Maulteile zu strecken und in die in Fig. 1 gezeigte gerade Form zu bringen. Durch diese elastische Verformung wird eine Vorspannkraft in den Halteabschnitten 16, 18 der Maulteile 6, 8 erzeugt, welche die beiden Greifabschnitte 12, 14 in Anlage hält.

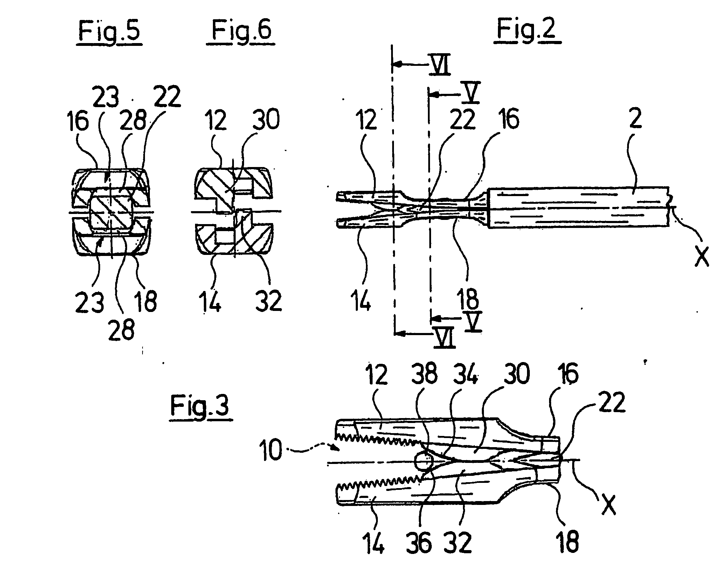

Zum Öffnen des Maules 10 weist das Instrument eine Betätigungsstange in Form einer Druckstange 20 auf. Die Druckstange 20 weist an ihrem distalen Ende ein keilförmig ausgebildetes Betätigungselement 22 auf. Das keilförmige Betätigungselement 22 verjüngt sich zum distalen Ende der Druckstange 20 hin und weist auf diese Weise zwei zueinander geneigte Keil- bzw. Druckflächen 24, 26 auf. Die Druckflächen 24, 26 sind so geneigt, dass sie an einer Spitze, welche auf der Instrumentenlängsachse X liegt, welche der Bewegungsrichtung der Druckstange 20 entspricht, einander berühren. Die Druckflächen 24, 26 kommen, wenn die Druckstange 20 in distaler Richtung vorgeschoben wird, mit den Innenflächen 28 (siehe Fig. 5) der Maulteile 6, 8 im Übergangsbereich zwischen den Halteabschnitten 16, 18 und den Greifabschnitten 12, 14 in Kontakt. Das keilförmige Betätigungselement 22 ist jeweils in einer Ausnehmung 23 des jeweiligen Halteabschnittes 16, 18 gelegen. Die Innenflächen 28, welche Betätigungsflächen bzw. Kanten bilden, stellen die distalen Stirnkanten dieser Ausnehmung 23 dar. Diese sind gerundet, sodass sie eine möglichst schmale Anlageflächen an den Druckflächen 24, 26 mit möglichst geringer Reibung bilden. So gleiten die Innen- bzw. Betätigungsflächen 28 auf den Druckflächen 24, 26. Auf diese Weise werden bei weiterem Vorschub der Druckstange 20 die Greifabschnitte 12, 14 in einer Richtung quer zur Instrumentenlängsachse X auseinander gedrückt und das Maul 10 geöffnet, wie in den Fig. 2, 3 und 4 gezeigt ist.

Im Bereich der Greifabschnitte 12, 14 sind zum proximalen Ende der Greifabschnitte 12, 14 hin gelegen an den Maulteilen 6, 8 Rückführungselemente 30, 32 ausgebildet. Bei diesen handelt es sich um nach innen, d. h. in Richtung auf das andere Maulteil zu, vorstehende Vorsprünge. Dabei sind die Rückführungselemente 30, 32 so angeordnet, dass sie in einer Richtung quer zur Instrumentenlängsachse X versetzt sind, sodass sie sich beim Schließen des Maules 10 aneinander vorbei bewegen können. Dabei überlappen sich die Rückführungselemente 30, 32 jedoch auch im geöffneten Zustand des Mauls. Idealerweise sind die beiden Maulteile 6, 8 identisch ausgebildet und das Rückführungselement 30 bzw. 32 ist jeweils aus der Mittelebene des Maulteiles 6 bzw. 8 seitlich versetzt, sodass es bei der Montage zweier solcher Maulteile gegenüberliegend zueinander zu dem beschriebenen Versatz der beiden Rückführungselemente 30, 32 kommt und diese aneinander vorbei bewegbar sind. Am distalen Ende weisen die Rückführungselemente 30, 32 schräg zur Instrumentenlängsachse X verlaufende Anlageflächen 34, 36 auf, welche dazu dienen, beim Schließen des Maules 10, wenn sich die Greifabschnitte 12, 14 aufeinander zu bewegen, z. B. eine zwischen den Maulteilen 6, 8 gelegenen Nadel 38 distalwärts in den Bereich der Greifabschnitte 12, 14 zu bewegen, in welcher sie sicher gehalten werden kann.

Die gelenklose Ausgestaltung der Maulteile 6, 8 hat den Vorteil, dass die Zahl der Einzelteile reduziert wird, die Reinigung vereinfacht wird und im Übrigen über den gesamten Öffnungsbereich des Maules 10 eine in etwa konstante Haltekraft gewährleistet werden kann.

Die Handhabe 4 ist wie das Maul 10 in diesem Beispiel symmetrisch zur Instrumentenlängsachse X ausgebildet und weist zwei Griffschenkel 40 auf, welche jeweils an einem Gelenkpunkt 42 an einer am proximalen Ende des Schaftes 2 ausgebildeten Griffaufnahme 44 gelenkig gelagert sind. Dabei sind die Gelenkpunkte 42 für die beiden Griffschenkel 40 in eine Richtung quer zur Instrumentenlängsachse X beabstandet. An ihrem proximalen Ende sind die Griffschenkel 40 jeweils mit einem Blattfederelement 46 verbunden. Die Verbindung zwischen den Griffschenkeln 40 und den Blattfederelementen 46 ist jeweils fest, d. h. nicht gelenkig. Die Blattfederelemente 46 sind so gekrümmt, dass sie sich in distaler Richtung erstrecken und dabei von den Innenflächen, d. h. den einander zugewandten Flächen der Griffschenkel 40 entfernen, sodass sich die beiden Blattfederelemente 46 in einem Anlenkungspunkt 48 treffen, an welchem sie gelenkig mit dem proximalen Ende der Druckstange 20 verbunden sind. Dabei sind die beiden Blattfederelemente 46 in einem gemeinsamen Anlenkungspunkt 48 mit der Druckstange 20 verbunden. Der Anlenkungspunkt 48 liegt auf der Instrumentenlängsachse X und ist in Richtung dieser Achse von den Gelenkpunkten 42 in proximaler Richtung beabstandet.

Zum Öffnen des Maules 10 werden die beiden Griffschenkel 40 zusammengedrückt, d. h. aufeinander zu bewegt, sodass sich bei der jeweiligen Schwenkbewegung um die Gelenkpunkte 42 die proximalen Enden der Griffschenkel 40 annähern. Bei dieser Schwenkbewegung werden die beiden Blattfederelemente 46 verformt, wobei sich aufgrund der festen Verbindung am proximalen Ende die distalen Enden der Blattfederelemente 46 mit dem Anlenkungspunkt 48 distalwärts verlagern, wodurch die Druckstange 20 in distaler Richtung vorgeschoben wird und das Betätigungselement 22 gegen die Betätigungsflächen 28 der Maulteile 6 und 8 drückt. Dabei werden dann die Maulteile 6 und 8 durch elastische Verformung geöffnet. Zum Schließen des Maules 10 werden die Griffschenkel 40 losgelassen, sodass sie sich aufgrund der Federwirkung der Blattfederelemente 46 wieder in ihre Ausgangslage zurück bewegen, d. h. auseinander schwenken. Dabei wird dann die Druckstange 20 in proximaler Richtung zurückgezogen. Aufgrund der oben beschriebenen elastischen Vorspannung der Maulteile 6 und 8 werden diese dann ohne weitere Sperrelemente sicher in Anlage gehalten, sodass beispielsweise eine Nadel 38 sicher zwischen den Greifabschnitten 12 und 14 geklemmt bleibt. Zum Lösen müssen dann lediglich die Griffschenkel 40 gedrückt werden, ohne dass weitere Sperr- oder Löseelemente betätigt werden müssten.

### Bezugszeichenliste

| | | |
|---|---|---|
| 2 | - | Schaft |
| 4 | - | Handhabe |
| 6, 8 | - | Maulteile |
| 10 | - | Maul |
| 12, 14 | - | Greifabschnitte |
| 16, 18 | - | Halteabschnitte |
| 20 | - | Druckstange |
| 22 | - | Betätigungselement |
| 23 | - | Ausnehmung |
| 24, 26 | - | Druckflächen |
| 28 | - | Innenflächen bzw. Betätigungsflächen |
| 30, 32 | - | Rückführungselemente |
| 34, 36 | - | Anlageflächen |
| 38 | - | Nadel |
| 40 | - | Griffschenkel |
| 42 | - | Gelenkpunkte |
| 44 | - | Griffaufnahme |
| 46 | - | Blattfederelemente |
| 48 | - | Anlenkungspunkt |
| | | |
| X | - | Instrumentenlüngsachse |

## Patentansprüche

1. Medizinisches Greifinstrument mit zwei Maulteilen (6, 8), welche einander gegenüberliegende distale Greifabschnitte (12, 14) aufweisen, wobei
zumindest eines der Maulteile (6, 8) einen proximalen Halteabschnitt (16, 18) aufweist, welcher derart elastisch verformbar ist, dass die distalen Greifabschnitte (12, 14) der beiden Maulteile (6, 8) durch elastische Verformung des zumindest einen Halteabschnittes (16, 18) aufeinander zu und voneinander weg bewegbar sind, die Greifabschnitte (12, 14) der beiden Maulteile (6, 8) in einer Ruhelage aneinander anliegen und durch elastische Verformung zumindest eines Halteabschnittes (16, 18) auseinanderbewegbar sind,
**dadurch gekennzeichnet, dass**
in der Ruhelage zumindest ein Halteabschnitt (16, 18) eines Maulteiles (6, 8) derart elastisch verformt ist, dass er eine Federkraft erzeugt, welche die Greifabschnitte (12, 14) der beiden Maulteile (6, 8) in Anlage hält, und dass ein keilförmiges Betätigungselement (22) vorhanden ist, welches linear in Richtung seiner Spitze derart zwischen die Maulteile (6, 8) bewegbar ist, dass die Greifabschnitte (12, 14) der beiden Maulteile (6, 8) unter elastischer Biegung zumindest eines Halteabschnittes (16, 18) auseinander bewegt werden.

2. Medizinisches Greifinstrument nach Anspruch 1, **dadurch gekennzeichnet, dass** beide Maulteile (6, 8) jeweils einen proximalen Halteabschnitt (16, 18)aufweisen, welcher derart elastisch verformbar ist, dass die Greifabschnitte (12, 14) der beiden Maulteile (6, 8) durch elastische Verformung beider Halteabschnitte (16, 18) aufeinander zu und voneinander weg bewegbar sind.

3. Medizinisches Greifinstrument nach einem der vorangehenden Ansprüche, **dadurch gekennzeichnet, dass** zur Bewegung des Greifabschnittes (12, 14) zumindest eines der Maulteile (6, 8) eine in Richtung der Instrumentenlängsachse X bewegliche Betötigungsstange (20) vorhanden ist, welche an ihrem proximalen Ende mit einer Handhabe (4) verbunden ist.

4. Medizinisches Greifinstrument nach einem der vorangehenden Ansprüche, **gekennzeichnet durch** eine Handhabe (4), welche zumindest einen Griffschenkel (40) aufweist, der an seinem distalen Ende gelenkig gelagert und an seinem proximalen Ende mit einem Blattfederelement (46) verbunden ist, welches sich ausgehend von dem proximalen Ende des Griffschenkels (40) distalwärts erstreckt und mit seinem distalen Ende beabstandet von dem Griffschenkel (40) mit dem proximalen Ende einer Betätigungsstange (20) verbunden ist.

5. Medizinisches Greifinstrument nach Anspruch 4, **dadurch gekennzeichnet, dass** die Anbindung des Blattfederelementes (46) an das proximale Ende der Betätigungsstange (20) in proximaler Richtung von der gelenkigen Lagerung (42) des Griffschenkels (40) beabstandet ist.

6. Medizinisches Greifinstrument nach einem der vorangehenden Ansprüche, **dadurch gekennzeichnet, dass** es als Nadelübernahmeinstrument ausgestaltet ist.

## Claims

1. A medical gripper instrument with two jaw parts (6, 8) which comprise distal gripping sections (12, 14) lying opposite one another, wherein
at least one of the jaw parts (6, 8) comprises a proximal holding section (16, 18) which is elastically deformable in a manner such that the distal gripping sections (12, 14) of the two jaw parts (6, 8) may be moved to one another and away from one another by way of elastic deformation of the at least one holding section (16, 18), the gripping sections (12, 14) of the two jaw parts (6, 8) bear on one another in an idle position and may be moved apart by way of elastic deformation of at least one holding section (16, 18), **characterised in that** in the idle position, at least one holding section (16, 18) of a jaw part (6, 8) is elastically deformed in a manner such that it produces a spring force which holds the gripping sections (12, 14) of the two jaw parts (6, 8) in bearing contact and that a wedge-like actuation element (22) is present, which is movable linearly in the direction of its tip, between the jaw parts (6, 8), in a manner such that the gripping sections (12, 14) of the two jaw parts (6, 8) are moved apart amid the elastic bending of at least one holding section (16, 18).

2. A medical gripper instrument according to claim 1, **characterised in that** both jaw parts (6, 8) in each case have a proximal holding section (16, 18) which is elastically deformable in a manner such that the gripping sections (12, 14) of the two jaw parts (6, 8) may be moved to one another and away from one another by way of elastic deformation of both holding sections (16, 18).

3. A medical gripper instrument according to one of the preceding claims, **characterised in that** an actuation rod (20) which is movable in the direction of the instrument longitudinal axis X and which at its proximal end is connected to a handle (4), is present for moving the gripping section (12, 14) at least of one of the jaw parts (6, 8).

4. A medical gripper instrument according to one of the preceding claims, **characterised by** a handle (4) which comprises at least one gripping limb (40) which is mounted in an articulated manner at its distal end and is connected at its proximal end to a leaf spring element (46) which extends distally departing from the proximal end of the gripping limb (40) and with its distal end distanced to the gripping limb (40), is connected to the proximal end of an actuation rod (20).

5. A medical gripper instrument according to claim 4, **characterised in that** the connection of the leaf spring element (46) to the proximal end of the actuation rod (20) is distanced in the proximal direction to the articulated mounting (42) of the gripping limb (40).

6. A medical gripper instrument according to one of the preceding claims, **characterised in that** it is designed as a needle take-over instrument.

## Revendications

1. Instrument médical de préhension comprenant deux parties de mâchoire (6, 8) qui présentent des segments de préhension distaux (12,14) opposés l'un à l'autre, dans lequel au moins une des parties de mâchoire (6, 8) présente un segment de retenue proximal (16, 18) qui peut se déformer élastiquement de façon telle que les segments de préhension distaux (12, 14) des deux parties de mâchoire (6, 8) peuvent se rapprocher et s'éloigner sous l'effet d'une déformation élastique du segment de retenue (16,18), au moins au nombre de un, les segments de préhension (12, 14) des deux parties de mâchoire (6, 8) reposant l'un contre l'autre dans une position de repos et pouvant s'écarter l'un de l'autre sous l'effet d'une déformation élastique d'au moins un segment de retenue (16, 18),
**caractérisé en ce que**,
dans la position de repos, au moins un segment de retenue (16, 18) d'une partie de mâchoire (6, 8) est déformé élastiquement de telle sorte qu'il génère une force élastique qui maintient les segments de préhension (12, 14) des deux parties de mâchoire (6, 8) en appui l'un contre l'autre et **en ce qu'**il est prévu un élément d'actionnement en forme de coin (22) qui peut être déplacé linéairement dans la direction de sa pointe entre les parties de mâchoire (6, 8) de telle manière que les segments de préhension (12, 14) des deux parties de mâchoire (6, 8) s'écartent l'un de l'autre par fléchissement élastique d'au moins un segment de retenue (16, 18).

2. Instrument médical de préhension selon la revendication 1, **caractérisé en ce que** les deux parties de mâchoire (6, 8) présentent chacune un segment de retenue proximal (16, 18) qui peut se déformer élastiquement de façon telle que les segments de préhension (12,14) des deux parties de mâchoire (6,8) peuvent se rapprocher et s'éloigner l'un de l'autre sous l'effet d'une déformation élastique des deux segments de retenue (16, 18).

3. Instrument médical de préhension selon l'une des revendications précédentes, **caractérisé en ce que** pour déplacer le segment de préhension (12, 14) d'au moins une des parties de mâchoire (6, 8), il est prévu une tige d'actionnement (20) qui est mobile dans la direction de l'axe longitudinal X de l'instrument et qui est reliée à une poignée (4) à son extrémité proximale.

4. Instrument médical de préhension selon l'une des revendications précédentes, **caractérisé par** une poignée (4) présentant au moins une branche de poignée (40) qui est montée de façon articulée à son extrémité distale et qui est reliée à son extrémité proximale à un élément formant ressort à lame (46) qui s'étend dans la direction distale en partant de l'extrémité proximale de la branche de poignée (40) et qui, à son extrémité distale éloignée de la branche de poignée (40), est reliée à l'extrémité proximale d'une tige d'actionnement (20).

5. Instrument médical de préhension selon la revendication 4, **caractérisé en ce que** la fixation de l'élément formant ressort à lame (46) à l'extrémité proximale de la tige d'actionnement (20) est éloignée de la structure articulée (42) de la branche de poignée (40) dans la direction proximale.

6. Instrument médical de préhension selon l'une des revendications précédentes, **caractérisé en ce qu'**il est exécuté en tant qu'instrument de saisie d'aiguille.
